# EUROPÄISCHE PATENTSCHRIFT

(11) **EP 3 160 538 B1**
(45) Veröffentlichungstag und Bekanntmachung des Hinweises auf die Patenterteilung: **21.04.2021**
(21) Anmeldenummer: 15732701.6
(22) Anmeldetag: 30.06.2015
(51) Int. Cl.: A61M 1/36

(54) **VORRICHTUNG ZUM ENTFERNEN VON FLUID AUS EINEM BLUTFILTER NACH BEENDIGUNG EINER BLUTBEHANDLUNGSSITZUNG MITTELS FLUSSERHÖHUNG**
DEVICE FOR REMOVING FLUID FROM A BLOOD FILTER AFTER COMPLETING A BLOOD TREATMENT SESSION BY MEANS OF FLOW INCREASE
DISPOSITIF POUR L'ÉLIMINATION DE FLUIDE À PARTIR D'UN FILTRE SANGUIN APRÈS LA FIN D'UNE SÉANCE DE TRAITEMENT DU SANG AU MOYEN D'UNE AUGMENTATION D'ÉCOULEMENT

(30) Priorität: 30.06.2014 DE 102014109136
(43) Veröffentlichungstag der Anmeldung: 03.05.2017
(73) Patentinhaber: Fresenius Medical Care Deutschland GmbH, 61352 Bad Homburg (DE)
(72) Erfinder: HÄCKER, Jürgen, 61267 Neu-Anspach (DE); GRONAU, Sören, 65428 Rüsselsheim (DE)
(74) Vertreter: Bobbert & Partner Patentanwälte PartmbB
(86) Internationale Anmeldenummer: PCT/EP2015/064833
(87) Internationale Veröffentlichungsnummer: WO 2016/001217

(56) Entgegenhaltungen:
- EP-A1- 1 539 271
- EP-A1- 2 535 065
- EP-A1- 2 745 864
- WO-A1-2010/121819
- US-A1- 2011 284 467

## Beschreibung

Die vorliegende Erfindung betrifft eine Steuerungsvorrichtung gemäß dem Oberbegriff des Anspruchs 1 zum Entfernen von Fluid aus einem Blutfilter für die extrakorporale Blutbehandlung eines Patienten nach Beendigung der Blutbehandlungssitzung. Des Weiteren betrifft die vorliegende Erfindung eine medizinische Behandlungsvorrichtung gemäß Anspruch 7, ein digitales Speichermedium gemäß Anspruch 11, ein Computerprogramm-Produkt gemäß Anspruch 12 sowie ein Computerprogramm gemäß Anspruch 13.

Blutfilter und extrakorporale Blutkreisläufe sind üblicherweise Einwegartikel und werden nach ihrer Verwendung entsorgt. Die Entsorgung ist kostenintensiv und nach Abfallgewicht zu vergüten. Aus diesem Grund, und auch zur Verringerung einer Kontaminationsgefahr, werden diese Einwegartikel daher vor ihrer Entsorgung von Fluid, und insbesondere von Blut, entleert. Ein Verfahren zur Entleerung eines Blutkreislaufs ist z.B. aus EP2535065 bekannt.

Es ist Aufgabe der vorliegenden Erfindung, ein weiteres Verfahren sowie geeignete Vorrichtungen zum Entfernen von Fluid aus einem Blutfilter oder einer Blutkammer hiervon und ggf. aus einem hiermit verbundenen extrakorporalen Blutkreislauf anzugeben.

Die erfindungsgemäße Aufgabe wird durch eine Steuerungseinrichtung mit den Merkmalen des Anspruchs 1 gelöst. Sie wird ferner gelöst mittels der medizinischen Behandlungsvorrichtung mit den Merkmalen des Anspruchs 7, des digitalen Speichermediums mit den Merkmalen des Anspruchs 11, des Computerprogramm-Produkts mit den Merkmalen des Anspruchs 12 sowie des Computerprogramms mit den Merkmalen des Anspruchs 13.

Erfindungsgemäß wird somit eine Steuerungseinrichtung zur Veranlassung eines Verfahrens zum Entfernen von Fluid, insbesondere Blut, aus einem zur Blutbehandlung eines Patienten verwendeten Blutfilter nach Beendigung der Blutbehandlungssitzung vorgeschlagen. Dabei weist der Blutfilter eine Blutkammer und eine Dialysatkammer auf, zwischen welchen eine Membran angeordnet ist, wobei die Blutkammer zur Blutbehandlung mit einer zur Blutkammer führenden arteriellen Blutleitung und einer von der Blutkammer wegführenden venösen Blutleitung sowie einer zur Dialysatkammer führenden Dialysierflüssigkeitszulaufleitung und einer von der Dialysatkammer wegführenden Dialysatablaufleitung verbunden ist. Das erfindungsgemäße Verfahren umfasst das Verdrängen des Fluids aus der Blutkammer durch Einbringen von Substituat oder Dialysierflüssigkeit in die arterielle Blutleitung, vorzugsweise stromaufwärts der Blutkammer.

Die erfindungsgemäße Steuerungseinrichtung, welche auch als Regelungseinrichtung ausgestaltet sein kann, ist geeignet und vorgesehen und/oder ausgelegt und/oder konfiguriert zur Durchführung des Verfahrens im Zusammenwirken mit einer medizinischen Blutbehandlungsvorrichtung. Sie kann gegebenenfalls weitere Einrichtungen wie beispielsweise Speichereinrichtungen, Zugabeeinrichtungen, automatisierte Signalgebungseinrichtungen usw. umfassen.

Die erfindungsgemäße medizinische Behandlungsvorrichtung (im Folgenden auch kurz: Behandlungsvorrichtung) weist wenigstens einen extrakorporalen Blutkreislauf mit einem Leitungsinneren auf. Ferner ist sie versehen mit wenigstens einer an oder in dem extrakorporalen Blutkreislauf angeordneten Blutpumpe zum Fördern von Blut innerhalb des Leitungsinneren des extrakorporalen Blutkreislaufs. Zudem weist sie wenigstens eine Einrichtung, etwa eine Fördereinrichtung oder Substituat- oder Dialysierflüssigkeitspumpe auf, welche vorgesehen ist zum Einbringen von Substituat oder Dialysierflüssigkeit in die arterielle Blutleitung. Zudem weist sie eine erfindungsgemäße Steuerungs- oder Regelungseinrichtung auf.

Ein erfindungsgemäßes, insbesondere digitales, insbesondere nicht-flüchtiges, Speichermedium (hier auch als Träger bezeichnet), insbesondere in Form von Diskette, RAM, ROM, CD, Festplatte, DVD, USB-Stick, Flashcard, SD-Karte oder EPROM, insbesondere mit elektronisch oder optisch auslesbaren Steuersignalen, kann derart mit einem programmierbaren Computer oder Computersystem zusammenwirken, dass die maschinellen Schritte eines hierin beschriebenen Verfahrens veranlasst werden. Dabei können alle, einige oder manche der maschinell durchgeführten Schritte des Verfahrens veranlasst werden.

Ein erfindungsgemäßes Computerprogramm-Produkt weist einen volatilen, flüchtigen oder auf einem maschinenlesbaren Träger gespeicherten Programmcode zur Veranlassung der maschinellen Schritte des Verfahrens, wenn das Computerprogramm-Produkt auf einem Rechner abläuft, auf.

Der Begriff "maschinenlesbarer Träger", wie er hierin verwendet wird, bezeichnet in bestimmten Ausführungsformen der vorliegenden Erfindung einen Träger, der von Software und/oder Hardware interpretierbare Daten oder Informationen enthält. Der Träger kann ein Datenträger, wie eine Diskette, eine CD, DVD, ein USB-Stick, eine Flashcard, eine SD-Karte, ein EPROM und dergleichen sein.

Ein erfindungsgemäßes Computerprogramm weist einen Programmcode auf zur Veranlassung der maschinellen Schritte des Verfahrens, wenn das Computerprogramm auf einem Computer abläuft.

Dabei können alle, einige oder manche der maschinell durchgeführten Schritte des Verfahrens veranlasst werden.

Unter einem Computerprogramm-Produkt kann erfindungsgemäß beispielsweise ein auf einem Träger gespeichertes Computerprogramm, ein Embedded System als umfassendes System mit einem Computerprogramm (z. B. ein elektronisches Gerät mit einem Computerprogramm), ein Netzwerk von computerimplementierten Computerprogrammen (z. B. Client/Serversystem, Cloud Computing System, etc.), oder ein Computer, auf dem ein Computerprogramm geladen ist, abläuft, gespeichert ist, ausgeführt oder entwickelt wird, verstanden werden.

Unter einem Computerprogramm kann erfindungsgemäß beispielsweise ein physikalisches, vertriebsfähiges Software-Produkt verstanden werden, welches ein Programm aufweist.

Auch für das erfindungsgemäße Computerprogramm-Produkt und das erfindungsgemäße Computerprogramm gilt, dass alle, einige oder manche der maschinell durchgeführten Schritte des Verfahrens veranlasst werden.

Bei allen folgenden Ausführungen ist der Gebrauch des Ausdrucks "kann sein" bzw. "kann haben" usw. synonym zu "ist vorzugsweise" bzw. "hat vorzugsweise" usw. zu verstehen und soll eine erfindungsgemäße Ausführungsform erläutern.

Wann immer hierin Zahlenworte genannt werden, so versteht der Fachmann diese als Angabe einer zahlenmäßig unteren Grenze.

Sofern dies zu keinem für den Fachmann erkennbaren Widerspruch führt, liest der Fachmann daher beispielsweise bei der Angabe "ein" oder "einem" stets "wenigstens ein" oder "wenigstens einem" mit. Dieses Verständnis ist ebenso von der vorliegenden Erfindung mit umfasst wie die Auslegung, dass ein Zahlenwort wie beispielsweise "ein" alternativ als "genau ein" gemeint sein kann, wo immer dies für den Fachmann erkennbar technisch möglich ist. Beides ist von der vorliegenden Erfindung umfasst und gilt für alle hierin verwendeten Zahlenworte.

Vorteilhafte Weiterentwicklungen der vorliegenden Erfindung sind jeweils Gegenstand von Unteransprüchen und Ausführungsformen.

In manchen erfindungsgemäßen Ausführungsformen dient das Verfahren dem teilweisen, in anderen dem vollständigen Entfernen von Blut aus einem zur Blutbehandlung eines Patienten verwendeten Blutfilter und/oder Blutkreislauf nach Beendigung der Blutbehandlungssitzung.

Der verwendete Blutfilter ist in einigen erfindungsgemäßen Ausführungsformen ein Hämodialysator.

Die zwischen Blutkammer und Dialysatkammer angeordnete Membran ist in bestimmten erfindungsgemäßen Ausführungsformen eine semipermeable Membran.

### In gewissen beispielhaften erfindungsgemäßen

Ausführungsformen führt die venöse Blutleitung von der Blutkammer des Blutfilters zu einer venösen Blutkammer (hierin auch als venöse Luftabscheidekammer bezeichnet) und/oder einer venösen Konnektionsstelle oder -einrichtung. In bestimmten beispielhaften erfindungsgemäßen Ausführungsformen führt die arterielle Blutleitung von einer arteriellen Konnektionsstelle oder -einrichtung bis zur Blutkammer des Blutfilters.

In erfindungsgemäßen Ausführungsformen umfasst das Verfahren das Verdrängen des Fluids aus der Blutkammer durch Einbringen von Substituat oder Dialysierflüssigkeit in die arterielle Blutleitung mit einem Fluss des Substituats oder der Dialysierflüssigkeit, welcher, zumindest phasenweise, über dem mittleren und/oder über dem maximalen Substituatfluss der vorangegangenen Blutbehandlung liegt.

In einigen beispielhaften erfindungsgemäßen Ausführungsformen umfasst das Verfahren das Verdrängen des Fluids aus der Blutkammer durch Einbringen von Substituat oder Dialysierflüssigkeit in die arterielle Blutleitung mit einem Fluss des Substituats oder der Dialysierflüssigkeit, welcher, zumindest phasenweise, über 150 ml/min, über 200 ml/min, über 250 ml/min, über 300 ml/min, über 350 ml/min, über 400 ml/min, über 450 ml/min, über 500 ml/min, über 550 ml/min und/oder gleich oder unter 600 ml/min liegt.

In manchen beispielhaften erfindungsgemäßen Ausführungsformen wird zum Verdrängen des Fluids aus der Blutkammer eingebrachtes Substituat oder eingebrachte Dialysierflüssigkeit mit einem ersten Fluss des Substituats oder der Dialysierflüssigkeit in einen Pufferbehälter hinein und mit einem zweiten Fluss aus dem Pufferbehälter heraus geführt. Der erste Fluss ist höher als der zweite.

### In gewissen beispielhaften erfindungsgemäßen

Ausführungsformen wird der erste Fluss des Substituats mittels Blutpumpe oder und/oder mittels Abgabe geeigneter Signale an die Blutpumpe erzeugt.

### In manchen beispielhaften erfindungsgemäßen Ausführungsformen wird der zweite Fluss mittels einer

Druckerzeugungsvorrichtung erzielt. Diese kann eine Pumpe, ein Kompressor oder dergleichen sein. Der Kompressor kann ein Luftkompressor sein. Die Druckerzeugungsvorrichtung kann angeordnet sein, um mittels Luft den im Pufferbehälter herrschenden Druck zu erhöhen, vorzugsweise so, dass das im Pufferbehälter vorliegende flüssige Fluid unter dem zweiten Fluss aus dem Pufferbehälter ausgetrieben wird.

### In gewissen beispielhaften erfindungsgemäßen

Ausführungsformen wird der zweite Fluss mittels einer Vorrichtung erzielt (vorzugsweise gesteuert oder geregelt), welche ein Ventil betätigt, welches eine Fluidkommunikation zwischen dem Inneren des Pufferbehälters und dem Äußeren des Pufferbehälters, je nach Ansteuerung, herstellt oder unterbindet. Der auf diese Weise hergestellte Druckausgleich kann zusammen mit der auf dem Fluid wirkenden Schwerkraft den gewünschten zweiten Fluss des Fluids bewirken.

In einigen beispielhaften erfindungsgemäßen Ausführungsformen ist der Pufferbehälter stromabwärts der Blutkammer angeordnet.

In bestimmten beispielhaften erfindungsgemäßen Ausführungsformen ist der Pufferbehälter eine Single-Needle-Kammer einer Blutkassette.

Eine solche Blutkassette ist beispielsweise in der WO 2010/121819 A1 der Anmelderin der vorliegenden Anmeldung beschrieben.

Eine solche Blutkassette kann wenigstens einen Gehäusekörper, vorzugsweise aus einem Hart-Kunstoff gefertigt, aufweisen, mit wenigstens einer, zwei oder mehreren in den Gehäusekörper integrierten Kammer(n) zum Aufnehmen medizinischer Fluide, wenigstens einem in den Gehäusekörper integrierten Kanal zum Aufnehmen und/oder Führen eines medizinischen Fluids; und wenigstens einer in den Gehäusekörper ganz oder teilweise integrierten Ventileinrichtung zum Steuern oder Regeln eines die Blutkassette durchströmenden Fluids.

Eine solche Blutkassette kann wenigstens eine Single-Needle-Kammer zum Aufnehmen von Blut stromab des Blutfilters aufweisen. Eine solche Single-Needle-Kammer kann wenigstens ein Single-Needle-Ventil umfassen oder hiermit in Fluidverbindung stehen, welches den Ein- und/oder Ausstrom vom Blut in die Single-Needle-Kammer steuert oder regelt. Beispiele für eine Single-Needle-Kammer und ein Single-Needle-Ventil sind beispielsweise in der WO 2010/121819 A1 beschrieben.

Die Single-Needle-Kammer kann - wie erfindungsgemäß jeder andere Pufferbehälter auch - mit einer Druckluftquelle verbunden sein. Sie kann hierzu vorzugsweise eine Sterilmembran aufweisen, durch welche hindurch mittels der Druckluftquelle Luft oder Sterilluft in die Single-Needle-Kammer eingebracht wird. Durch die mit dem Einbringen von Sterilluft in die Single-Needle-Kammer bewirkte Druckerhöhung (in einem oberen Bereich der Single-Needle-Kammer) wird das (sich in einem unteren Bereich der Single-Needle-Kammer befindende) Fluid aus der Single-Needle-Kammer ausgebracht. Das Fluid kann bei entsprechender Steuerung oder Regelung der Druckluftquelle und/oder Betätigung von entsprechenden Ventilen mit dem zweiten Fluss ausgebracht werden. Der zweite Fluss kann dabei auf den gewünschten Reinfusionsfluss eingestellt werden. Dieser kann dabei vom ersten Fluss, welcher in die Single-Needle-Kammer hinein führt, entkoppelt werden.

Eine solche Blutkassette kann an wenigstens einer ihrer Oberflächen eine Abdeckungseinrichtung aufweisen, welche vorzugsweise Teil wenigstens einer integrierten Ventileinrichtung ist.

Bei einer solchen Blutkassette kann die Abdeckungseinrichtung wenigstens in einem Abschnitt mit dem Gehäusekörper kraft- und/oder form- und/oder stoffschlüssig verbunden sein.

Bei einer solchen Blutkassette kann die Abdeckungseinrichtung mittels wenigstens einer umlaufenden Schweißnaht mit dem Gehäusekörper verbunden sein.

In manchen beispielhaften erfindungsgemäßen Ausführungsformen ist die Steuerungs- und/oder Regelungseinrichtung konfiguriert, um im Zusammenwirken mit einer medizinischen Blutbehandlungsvorrichtung eine Blutbehandlung und eine sich anschließende Verdrängung des Fluids, insbesondere Bluts, aus der Blutkammer mittels eingebrachtem Substituat oder eingebrachter Dialysierflüssigkeit mit einem vorbestimmten, vorzugsweise schwankenden oder nicht-konstanten Fluss hiervon, beispielsweise durch den Blutfilter, beispielsweise durch Abgabe eines entsprechenden Signals an eine Blutpumpe, zu bewirken. Der hier als vorbestimmt bezeichnete Fluss kann einen Fluss an eingebrachtem Substituat oder eingebrachter Dialysierflüssigkeit, beispielsweise in den Patienten erzeugen, welcher im Mittel 150 ml/min, ggf. +/- 30%, 20% oder 10% hiervon, beträgt. Das Mittel kann sich über die gesamte Dauer der Blutbehandlung einschließlich der sich anschließenden Verdrängung des Fluids beziehen.

In einigen beispielhaften erfindungsgemäßen Ausführungsformen weist die medizinische Behandlungsvorrichtung einen Pufferbehälter zum Aufnehmen von Fluid (pur oder gemischt), welches aus der Blutkammer verdrängt wurde, auf oder ist hiermit, vorzugsweise in Fluidkommunikation, verbunden. Die erfindungsgemäße Steuervorrichtung kann konfiguriert sein, um den Pufferbehälter im Zusammenwirken mit Pumpen, Klemmen oder anderen Einrichtungen als solchen zu nutzen.

In einigen beispielhaften erfindungsgemäßen Ausführungsformen ist der Pufferbehälter stromabwärts der Blutkammer angeordnet.

### In gewissen beispielhaften erfindungsgemäßen

Ausführungsformen sind die medizinische Behandlungsvorrichtung und/oder der Blutfilter mit einer Blutkassette verbunden, wobei der Pufferbehälter eine Single-Needle-Kammer der Blutkassette ist.

In bestimmten beispielhaften erfindungsgemäßen Ausführungsformen wird wenigstens eine Patientenklemme zum Unterbinden einer Strömung von Blut innerhalb der arteriellen Blutleitung und/oder der venösen Blutleitung geschlossen. In einigen Ausführungsformen wird zusätzlich auch ein Single-Needle-Ventil geschlossen.

In manchen beispielhaften erfindungsgemäßen Ausführungsformen wird eine arterielle Blutleitung mit einer venösen Blutleitung verbunden zum Zwecke der Entleerung des Blutfilters. In gewissen erfindungsgemäßen Ausführungsformen unterbleibt eine Verbindung zwischen arterieller Blutleitung und venöser Blutleitung völlig.

In einigen beispielhaften erfindungsgemäßen Ausführungsformen wird die Verbindung zwischen arterieller Blutleitung und venöser Blutleitung insbesondere ausschließlich zum Zwecke der Entleerung des Blutfilters erzeugt.

In einigen beispielhaften erfindungsgemäßen Ausführungsformen wird eine qualitative Änderung des Inhalts des Leitungsinneren des extrakorporalen Blutkreislaufs erfasst.

In manchen erfindungsgemäßen Ausführungsformen wird durch Betreiben einer Fördereinrichtung, etwa der Blutpumpe, der Substituatpumpe oder der Dialysierflüssigkeitspumpe, eine vorbestimmte Menge an Substituat oder Dialysierflüssigkeit in das Leitungsinnere des extrakorporalen Blutkreislaufs eingebracht.

In einigen erfindungsgemäßen Ausführungsformen wird Substituat (alternativ als Substituatflüssigkeit zu bezeichnen) oder Dialysierflüssigkeit gefördert, bis die Erfassungseinrichtung in vorbestimmtem Maße Substituat oder Dialysierflüssigkeit im Leitungsinneren des extrakorporalen Blutkreislaufs erfasst.

In manchen erfindungsgemäßen Ausführungsformen ist die Erfassungsvorrichtung mit vorgegebener Distanz zu einer venösen Zugangseinrichtung angeordnet. Das Verfahren umfasst in diesen Ausführungsformen ferner das Fördern des Inhalts des Leitungsinneren des extrakorporalen Blutkreislaufs über die vorgegebene Distanz bis zur venösen Zugangseinrichtung, nachdem Dialysat an der Erfassungseinrichtung erkannt wurde.

In bestimmten erfindungsgemäßen Ausführungsformen wird im Leitungsinneren des extrakorporalen Blutkreislaufs enthaltenes Blut über die venöse Zugangseinrichtung in das Gefäßsystem des Patienten eingebracht.

Das Verfahren umfasst in einigen erfindungsgemäßen Ausführungsformen ferner das Eintragen oder Einbringen von Luft und Substituat in den extrakorporalen Blutkreislauf, beispielsweise nach Beenden einer Blutbehandlungssitzung.

Eine "Blutbehandlungssitzung" kann beispielsweise eine Behandlungseinheit mittels Hämodialyse, Hämofiltration, Hämodiafiltration und/oder eines Zellseparationsverfahrens und auf die Behandlung und/oder Reinigung von Blut gerichtet sein. Zum Durchführen einer solchen Blutbehandlung wird eine geeignete Blutbehandlungsvorrichtung verwendet.

Eine zum Durchführen des Verfahrens geeignete Blutbehandlungsvorrichtung weist in manchen erfindungsgemäßen Ausführungsformen einen extrakorporalen Blutkreislauf mit einem Leitungsinneren, wenigstens eine Fördereinrichtung zum Einbringen und/oder Fördern wenigstens zweier Fluide im Leitungsinneren des extrakorporalen Blutkreislaufs, und beispielsweise eine Einrichtung zum Behandeln des Bluts des Patienten, wie einen oder mehrere Blutfilter und/oder einen oder mehrere Dialysatoren und/oder einen oder mehrere Adsorber, auf oder ist hiermit verbunden. Sie kann ferner Behälter zum Aufbewahren von Fluiden, Elemente zum Einbringen der Fluide, wie beispielsweise Schlauchelemente und/oder Ventile, sowie weitere Einrichtungen, wie z. B. eine Luftabscheidekammer zum Entfernen von Luft aus dem Blut während der Blutbehandlung und/oder Sensoren und/oder Detektoren zum Erfassen verschiedener relevanter Parameter, wie beispielsweise einen Druck im extrakorporalen Blutkreislauf, aufweisen. Fördereinrichtungen, wie hierin genannt, schließen Membranpumpen, Schlauchpumpen, Rollenpumpen usw. ein. Die Blutpumpe, eine Substituatpumpe und/oder eine Dialysierflüssigkeitspumpe können z. B. als Schlauchpumpe oder Rollenpumpe ausgeführt sein. Es können aber auch ein anderer Pumpentyp, z. B. eine Membranpumpe, insbesondere eine hochgenau dosierende Membranpumpe, eingesetzt werden.

Bei einer Fördereinrichtung für Dialysierflüssigkeit oder Substituat kann es sich um eine "zweite" Fördereinrichtung handeln, also eine Fördereinrichtung, welche nicht mit der Blutpumpe identisch ist. Die Blutpumpe kann jedoch auch derart ausgestaltet sein, dass sie sowohl die für eine Blutpumpe typische Funktion ausführt als auch die Funktion des Einbringens von Substituat in das Leitungsinnere und/oder das Fördern von Leitungsinhalt erfüllt. Wenn im Folgenden allein der besseren Lesbarkeit wegen von einer Fördereinrichtung zum Einbringen von Substituat oder Dialysierflüssigkeit die Rede ist, so ist darunter die Blutpumpe oder eine sich hiervon unterscheidende Fördereinrichtung zu verstehen. Beide Varianten sind gleichermaßen von der vorliegenden Erfindung umfasst.

In erfindungsgemäßen Ausführungsformen wird der Substituatfluss nach Abschluss der Behandlung und/oder zum Freispülen des Blutfilters von Blut auf einen Wert erhöht, welcher über jenem oder jedem Wert liegt, welcher während der Blutbehandlung galt. Um die mittlere Reinfusionsgeschwindigkeit zu begrenzen folgen in diesen Ausführungsformen Hochflussphasen derart Niedrigflussphasen, dass sich im Mittel der gewünschte Reinfusionsvolumenstrom von z.B. 150 ml/min einstellt.

Das Verfahren umfasst in gewissen erfindungsgemäßen Ausführungsformen den Schritt des Einbringens oder Eintragens von Luft in das Leitungsinnere des extrakorporalen Blutkreislaufs, um diesen von Flüssigkeit zu entleeren, durch Betreiben der Blutpumpe. Die Luft kann beispielsweise Atmosphärenluft sein. Die vorliegende Erfindung soll jedoch nicht auf die alleinige Verwendung von Luft beschränkt sein, sondern ferner alle, für die Zwecke der vorliegenden Erfindung geeigneten, gasförmigen Fluide anstelle von Luft einschließen.

Das "Eintragen von Luft in das Leitungsinnere des extrakorporalen Blutkreislaufs" nach Beenden der Blutbehandlungssitzung kann ausschließlich oder unterstützend durch die Blutpumpe, durch die zweite Fördereinrichtung oder durch eine Druckluftquelle erfolgen.

Kombinationen der vorgenannten Optionen sind erfindungsgemäß ebenfalls umfasst, ebenso ein passives Eindringenlassen von Luft.

Das "Einbringen von Substituat oder Dialysierflüssigkeit in das Leitungsinnere des extrakorporalen Blutkreislaufs" erfolgt, wie vorstehend ausgeführt, in einigen erfindungsgemäßen Ausführungsformen durch Betreiben der Blutpumpe und/oder der Fördereinrichtung.

Die Blutpumpe kann Substituat fördern, indem sie selbige aus einer Zuleitung zu einem Behälter für das Substituat ansaugt, die stromaufwärts der Saugseite der Blutpumpe in den extrakorporalen Blutkreislauf mündet. Hierzu kann beispielsweise eine im arteriellen Zweig des extrakorporalen Blutkreislaufs vorgesehene Mündung mit Schlauchklemme vorgesehen sein.

Ist vorgesehen, dass die Blutpumpe sowohl Blut als auch Substituat in den extrakorporalen Blutkreislauf einbringt und fördert, kann das Verfahren mit nur einer Pumpe durchgeführt werden. Obwohl eine solche weiter bevorzugte Ausführungsform von der vorliegenden Erfindung umfasst ist, werden im Folgenden Ausführungsformen beschrieben, bei denen eine Blutpumpe und eine zweite Fördereinrichtung eingesetzt werden. Die folgende Beschreibung soll ein Verständnis der der vorliegenden Erfindung zugrunde liegenden Prinzipien und Funktionen der einzelnen Komponenten vereinfachen.

Ein "Substituat" kann dabei beispielsweise jedes zur Verwendung bei einer Blutbehandlung, wie z. B. einer Hämodiafiltration, allgemein bekannte Substituat oder Dialysierflüssigkeit sein, vorzugsweise eine bereits während der Blutbehandlungssitzung verwendete und somit über eine Fluidverbindung bereits in den extrakorporalen Blutkreislauf eingebrachte oder einbringbare Lösung oder isotone Kochsalzlösung, wie z. B. eine 0,9%-ige NaCl-Lösung.

In einer bevorzugten erfindungsgemäßen Ausführungsform ist das Erfassen einer qualitativen Änderung des Inhalts des Leitungsinneren des extrakorporalen Blutkreislaufs mit Hilfe wenigstens einer in oder an einem Abschnitt des extrakorporalen Blutkreislaufs angeordneten Erfassungseinrichtung umfasst.

Die "qualitative Änderung" kann sich auf einen oder mehrere Bereiche oder Abschnitte des extrakorporalen Blutkreislaufs beziehen, beispielsweise einen Bereich oder Abschnitt, in welchem die Erfassungseinrichtung vorliegt.

Eine "qualitative Änderung des Inhalts des Leitungsinneren" schließt eine Änderung der Zusammensetzung des Inhalts des Leitungsinneren, wie beispielsweise eine Änderung der einzelnen Anteile an Blut und/oder Substituat im Leitungsinneren oder einem Abschnitt hiervon, bezogen aufeinander, ein. Auch das Fehlen eines vormals vorhandenen Fluids kann eine Änderung der Zusammensetzung darstellen. Eine qualitative Änderung kann auch ein Übergang von Blut zu Substituat sein. Solche Änderungen können z. B. leicht anhand einer optischen Änderung des Inhalts, wie einer Aufhellung oder einer Verdunkelung des Inhalts, erfasst werden.

Die in einem Abschnitt des extrakorporalen Blutkreislaufs angeordnete "Erfassungseinrichtung" kann z. B. ein optischer Sensor sein, der eine optische Änderung des Inhalts des Leitungsinneren oder eine Eigenschaft des Inhalts erfasst. Weitere geeignete Sensoren schließen Drucksensoren, Leitfähigkeitssensoren und Sensoren zum Erfassen einer Änderung der Dichte des Inhalts des Leitungsinneren des extrakorporalen Blutkreislaufs ein, ohne darauf beschränkt zu sein.

Der "Abschnitt des extrakorporalen Blutkreislaufs" kann ein arterieller und/oder venöser Abschnitt des extrakorporalen Blutkreislaufs sein. Der "arterielle Abschnitt" bezeichnet einen Abschnitt des extrakorporalen Blutkreislaufs, durch den das Blut aus dem Gefäßsystem des Patienten in Richtung zur Blutbehandlungseinrichtung oder zum Blutfilter strömt. Der "venöse Abschnitt" bezeichnet den Abschnitt des extrakorporalen Blutkreislaufs, durch den das Blut aus der Blutbehandlungseinrichtung oder aus dem Blutfilter zurück zum Gefäßsystem des Patienten strömt.

In einer ebenfalls bevorzugten Weiterentwicklung des Verfahrens umfasst der extrakorporale Blutkreislauf wenigstens eine mit einem Abschnitt des Gefäßsystems des Patienten konnektierbare Zugangseinrichtung, und das Verfahren umfasst ein Dekonnektieren des extrakorporalen Blutkreislaufs vom Gefäßsystem des Patienten, insbesondere im Bereich einer ersten, beispielsweise arteriellen, Zugangseinrichtung, insbesondere an einem Ende des extrakorporalen Blutkreislaufs.

Das "Dekonnektieren des extrakorporalen Blutkreislaufs vom Gefäßsystem des Patienten" bedeutet das Unterbrechen einer Verbindung zwischen dem extrakorporalen Blutkreislauf und dem Gefäßsystem des Patienten in einem Abschnitt des extrakorporalen Blutkreislaufs, beispielsweise an einem Ende hiervon. Dabei kann die Unterbrechung sowohl am arteriellen als auch am venösen Abschnitt erfolgen, wobei in der vorliegenden Erfindung ein Dekonnektieren des arteriellen Abschnitts des extrakorporalen Blutkreislaufs bevorzugt ist.

Unter einem Dekonnektieren im "Bereich der ersten Zugangseinrichtung" kann z. B. das Herausziehen der arteriellen Konnektionsnadel eines Double-Needle-Zugangs verstanden werden.

Unter einem Dekonnektieren kann auch das Unterbrechen der Strömungsverbindung zwischen dem arteriellen Abschnitt des extrakorporalen Blutkreislaufs und der arteriellen Konnektionsnadel verstanden werden.

Bei der Single-Needle-Variante kann das Unterbrechen der Verbindung zwischen dem arteriellen Schenkel des "Y"-förmigen Abschnitts des extrakorporalen Blutkreislaufs und der einzigen mit dem Gefäßsystem des Patienten verbundenen Konnektionsnadel verstanden werden. Das offene Lumen des arteriellen Schenkels des Y-Stücks kann nach seinem Abtrennen auf beliebige Weise (manuell, maschinell, automatisch, usw.) verschlossen werden.

Alternativ oder zusätzlich kann gleiches auch für den venösen Abschnitt des extrakorporalen Blutkreislaufs und den venösen Zugang zum Gefäßsystem des Patienten angewandt werden.

Eine "Zugabestelle des extrakorporalen Blutkreislaufs für Substituat in das Leitungsinnere des extrakorporalen Blutkreislaufs" kann in dem arteriellen und/oder dem venösen Abschnitt des extrakorporalen Blutkreislaufs angeordnet sein. Bevorzugt ist die "Zugabestelle" in einem Abschnitt des extrakorporalen Blutkreislaufs angeordnet, der stromaufwärts der Blutbehandlungseinrichtung, wie beispielsweise des Blutfilters, durchströmt wird.

Geeignete Beispiele für eine Zugabestelle schließen ein Öffnungs-/Verschlussventil, einen Absperrhahn, eine zuschaltbare Nebenleitung eines verzweigten Abschnitts des extrakorporalen Blutkreislaufs usw. ein.

Eine "vorbestimmte Substituatsmenge oder Dialysierflüssigkeitsmenge" kann einem bestimmten Fördervolumen und/oder einer bestimmten Förderstrecke des Inhalts entlang des Leitungsinneren des extrakorporalen Blutkreislaufs entsprechen und beispielsweise durch Betreiben einer hochgenau dosierenden Membranpumpe erfolgen.

Die Substituatsmenge oder Dialysierflüssigkeitsmenge kann vorzugsweise als Größe, zum Beispiel als Volumen mit vorgegebener Maßzahl und Einheit, vorbestimmt sein. Die absolute Größe der Substituatsmenge kann vorzugsweise beispielsweise in einer Steuereinrichtung der erfindungsgemäßen Behandlungsvorrichtung hinterlegt und/oder eingebbar sein. Die Substituatsmenge kann vorzugsweise im Rahmen der technischen Genauigkeit exakt gefördert werden.

Um eine exakte Menge an Substituat vorzubestimmen, können zum Beispiel technische Spezifikationen des eingesetzten extrakorporalen Blutkreislaufs, wie beispielsweise die Innenvolumina des Schlauchsatzes, in der Steuereinrichtung gespeichert oder in diese eingegeben werden. Anhand der technischen Spezifikationen der einzelnen Komponenten des extrakorporalen Blutkreislaufs können zum Beispiel eine erforderliche Förderzeit und/oder ein Fördervolumen berechnet werden.

Eine "begrenzte Substituatsmenge oder Dialysierflüssigkeitsmenge" kann eine, zum Beispiel anhand von Erfahrungswerten des Bedienpersonals ausgewählte, Menge an Substituat oder Dialysierflüssigkeit sein. Vorzugsweise kann eine begrenzte Menge eingebracht und so lange gefördert werden, bis an einer weiteren Erfassungseinrichtung das Auftreten von Substituat im Leitungsinneren des extrakorporalen Blutkreislaufs erfasst wird. Eine begrenzte Substituatsmenge muss daher nicht genau bekannt sein und/oder einem bestimmten Fördervolumen entsprechen. Eine begrenzte Substituatsmenge kann jedoch indirekt durch das Innenvolumen der von der Substituatsmenge durchströmten Komponenten des extrakorporalen Blutkreislaufs, insbesondere das Innenvolumen des Abschnitts von der Zugabestelle für Substituat und/oder der Blutbehandlungseinrichtung bis zu einer weiteren Erfassungseinrichtung begrenzt sein. Das Volumen ist dabei somit bestimmt im Sinne von "begrenzt", ohne jedoch exakt bekannt zu sein, und ohne beispielsweise in Millilitern angegeben werden zu können und/oder ohne in einer Steuerung hinterlegt worden oder eingebbar zu sein. Das Einbringen einer begrenzten Substituatsmenge kann zum Beispiel von Vorteil sein, wenn der Filtertyp einer Blutbehandlungseinrichtung oder deren Fassungsvermögen nicht bekannt oder nicht richtig angegeben ist.

Dabei kann das Substituat aus einem dafür vorgesehenen Vorratsbehälter über entsprechende Leitungssysteme des extrakorporalen Blutkreislaufs an der Zugabestelle für das Substituat in den extrakorporalen Blutkreislauf eingebracht werden.

Die "Erfassungseinrichtung" ist wie vorstehend definiert und kann beispielsweise im venösen Abschnitt des extrakorporalen Blutkreislaufs, z. B. zwischen der Blutbehandlungseinrichtung und der venösen Zugangseinrichtung zum Gefäßsystem des Patienten und insbesondere zwischen einer Tropfkammer im venösen Abschnitt und der venösen Zugangseinrichtung, angeordnet sein.

Die Erfassungseinrichtung kann das Auftreten von Substituat in einem bestimmten Abschnitt des Leitungsinneren des extrakorporalen Blutkreislaufs beispielsweise anhand einer optischen Änderung des Inhalts des Leitungsinneren erkennen.

Wenn die Erfassungseinrichtung das Auftreten von Luft oder Substituat in dem Leitungsinneren des extrakorporalen Blutkreislaufs erkennt, kann das Fördern des "Substituat-Blut-Inhalts" gestoppt werden.

Dies kann durch Stoppen der entsprechenden Fördereinrichtung erfolgen.

Ferner ist in einer weiteren Ausführungsform des Verfahrens bevorzugt, die Erfassungseinrichtung mit vorgegebener Distanz zu einer zweiten Zugangseinrichtung anzuordnen und den Inhalt des Leitungsinneren über die vorgegebene Distanz bis zur Zugangseinrichtung zu fördern, nachdem Substituat oder eine vorbestimmte Transmission an der Erfassungseinrichtung erkannt wurde.

In einer weiter bevorzugten Ausführungsform des Verfahrens wird das im Leitungsinneren des extrakorporalen Blutkreislaufs enthaltene Blut - insbesondere im Wesentlichen vollständig - über die zweite Zugangseinrichtung in das Gefäßsystem des Patienten rückgeführt. Der Begriff "im Wesentlichen vollständig rückgeführt" meint hierbei, dass das im Leitungsinneren des extrakorporalen Blutkreislaufs vorhandene Blut nahezu rückstandsfrei aus dem extrakorporalen Blutkreislauf entfernt wird. Die gegebenenfalls im extrakorporalen Blutkreislauf aus technischen Gründen wie Benetzungsverhalten oder in der Tropfkammer verbleibenden Blutrückstände sind dabei als vernachlässigbar gering anzusehen.

Das "Rückführen von Blut in das Gefäßsystem des Patienten" kann erfolgen, wenn ein Ende des extrakorporalen Blutkreislaufs, wie beispielsweise das Ende des venösen Abschnitts, z. B. die venöse Konnektionsnadel, mit dem Gefäßsystem des Patienten konnektiert ist. Diese Verbindung kann nach Beenden der Blutbehandlungssitzung beibehalten oder erneut hergestellt werden.

Da mit der erfindungsgemäßen Behandlungsvorrichtung das vorstehend beschriebene Verfahren durchführbar ist, wird zur Vermeidung von Wiederholungen auf die vorstehend beschriebenen Ausführungen derselben verwiesen.

Eine Weiterentwicklung der erfindungsgemäßen Behandlungsvorrichtung sieht das Anordnen wenigstens einer Erfassungseinrichtung zum Erfassen wenigstens einer Änderung des Inhalts des Leitungsinneren des extrakorporalen Blutkreislaufs oder einer Eigenschaft des Inhalts in einem Abschnitt des extrakorporalen Blutkreislaufs vor. Bei einer Eigenschaft des Inhalts kann es sich um eine Zusammensetzung, eine physikalische, chemische oder biologische Größe, beispielsweise eine Transparenz, einen pH-Wert und vieles dergleichen mehr handeln. Eine derartige Erfassungseinrichtung kann der vorstehend beschriebenen entsprechen, so dass zur Vermeidung von Wiederholungen auf deren obige Beschreibung verwiesen wird.

Eine Behandlungsvorrichtung gemäß der vorliegenden Erfindung kann, ohne darauf beschränkt zu sein, zum Durchführen einer Hämodialyse, einer Hämofiltration, einer Hämodiafiltration und Separationsverfahren geeignet und/oder konfiguriert sein.

Mittels mancher erfindungsgemäßer Ausführungsformen können eine oder mehrere der hierin genannten Vorteile erzielbar sein.

So kann die vorliegende Erfindung in bestimmten erfindungsgemäßen Ausführungsformen zu einer Verbesserung der Effektivität der AV-Blutrückgabe beitragen.

Ein erfindungsgemäß in einigen Ausführungsformen erzielbarer Vorteil besteht darin, dass einem Kontaminationsrisiko bei der weiteren Handhabung oder der Entsorgung des Blutfilters und/oder des extrakorporalen Blutkreislaufs vorgebeugt oder dieses verringert werden kann, da nach Abschluss des erfindungsgemäßen Verfahrens kein Blut mehr im Blutfilter verbleibt oder dessen Menge merklich verringert wurde.

Ferner kann das Bruttoabfallgewicht des Blutfilters und/oder des extrakorporalen Blutkreislaufs verringert werden. Da eine Entsorgung von kontaminierten Einwegartikeln wie Blutfilter und extrakorporalem Blutkreislauf nach Gewicht berechnet wird, können somit in vorteilhafter Weise Kosten eingespart werden.

Die Entleerung wenigstens des Blutfilters, insbesondere von Blut, kann in bestimmten Ausführungsformen der Erfindung ohne wesentliche oder ohne jede Interaktion des Benutzers, z. B. des Arztes, erfolgen, jedenfalls bis zur Entnahme des Blutfilters von der Behandlungsvorrichtung. Hiermit einher geht, dass die Fehleranfälligkeit niedrig ist. Zudem hat der Benutzer während der Entleerung Zeit für andere Tätigkeiten. Dies trägt zur Arbeitsentlastung und zur Zeiteinsparung insgesamt bei.

Die vorliegende Erfindung kann ferner in vorteilhafter Weise zum sicheren Entfernen auch von Blut aus dem extrakorporalen Blutkreislauf für eine Behandlungsvorrichtung zur extrakorporalen Blutbehandlung eines Patienten nach Beenden einer Blutbehandlungssitzung eingesetzt werden: Da sich Substituat im Leitungsinneren des extrakorporalen Blutkreislaufs nach Beenden der Blutbehandlungssitzung befindet, kann das im Leitungsinneren des extrakorporalen Blutkreislaufs vorhandene Blut aus dem extrakorporalen Blutkreislauf entfernt werden. Dies kann ohne Gefahr erfolgen, Luft in den Körper des Patienten einzubringen.

Das Einbringen von Substituat unter erhöhtem Druck bzw. Fluss wie oben beschrieben kann ferner dazu beitragen, einen Blutfilter mit verschiedenen Kapillarinnendurchmessern von Blut zu reinigen, was beispielsweise durch ledigliches Durchströmen mit Luft aufgrund des geringeren Druckabfalls über den bereits luftgefüllten Kapillaren im Gegensatz zu den noch mit Blut gefüllten Kapillaren technisch schlecht realisierbar wäre.

Daneben kann es auch möglich sein, eine Schaumbildung, z. B. am Ausgang des Blutfilters, zu minimieren, indem dort Substituat, also insbesondere eine Flüssigkeit, nicht aber ein Gas (z. B. Luft) zum Verdrängen von Blut oder Entleeren eingesetzt wird.

Da der übliche, mittlere Reinfusionsfluss von beispielsweise 150 ml/min erfindungsgemäß beim Freispülen nicht überstiegen wird, werden das Wohlbefinden des Patienten und die Unversehrtheit des Gefäßsystems und insbesondere eines Shunts des Patienten durch das erfindungsgemäße Freispülen nicht beeinträchtigt. Zwar können erfindungsgemäß deutlich höhere Freispülflüsse als die vorgenannten 150 ml/min auftreten. Jedoch entspricht dieser Freispülfluss in bestimmten erfindungsgemäßen Ausführungsformen aufgrund der Pufferwirkung des Pufferbehälters und der Tatsache, dass der Ausstrom hieraus geringer ist als der Einstrom in den Pufferbehälter hinein, nicht der Reinfusionsgeschwindigkeit oder dem Reinfusionsfluss, mit welchem Blut dem Patienten reinfundiert wird.

Die Erfinder der vorliegenden Erfindung haben erkannt, dass die Effektivität des Ausspülens des Bluts aus dem Blutfilter mit steigendem Fluss zunimmt. Dies wird erfindungsgemäß in bestimmten Ausführungsformen erreicht.

Da das Verfahren direkt nach Beenden einer Blutbehandlungssitzung durchgeführt werden kann, ist es einfach und leicht durchzuführen und erfordert keine technisch aufwändigen, zeit- und/oder kostenintensiven Schritte.

Das Verfahren kann in vorteilhafter Weise mit der bei einer Blutbehandlung ohnehin eingesetzten oder vorliegenden Substituat- oder Dialysierflüssigkeit, wie beispielsweise einer isotonen Kochsalzlösung, z. B. einer 0,9%-igen NaCl-Lösung, durchgeführt werden. Dies trägt wiederum in vorteilhafter Weise zu einer Kosten- und Zeitersparnis bei.

Ferner kann das Verfahren ein Entfernen von Blut aus dem arteriellen Abschnitt des extrakorporalen Blutkreislaufs und insbesondere aus der arteriellen Konnektionsnadel und ein Rückführen desselben in das Gefäßsystem des Patienten ermöglichen. Es kann somit in vorteilhafter Weise der Schritt umgangen werden, das in der arteriellen Konnektionsnadel befindliche Blut mit Hilfe z. B. einer mit Kochsalzlösung aufgezogenen Spritze retrograd herauszudrücken.

Das Verfahren kann somit den Vorteil bieten, das im Leitungsinneren eines extrakorporalen Blutkreislaufs nach dessen Verwendung bei einer Blutbehandlung vorhandene Blut im Wesentlichen vollständig für den Patienten zurückzugewinnen.

Die erfindungsgemäße Vorgehensweise kann sicherstellen, dass beim Entleeren keine Luft in das Gefäßsystem des Patienten eintritt. Ferner kann es bei diesem Verfahren zu keiner Schaumbildung im Bereich eines im extrakorporalen Blutkreislauf vorhandenen Blutfilters kommen, was ein Entleeren des Bluts aus dem extrakorporalen Blutkreislauf erschweren würde. Im Blutfilter oder im extrakorporalen Blutkreislauf verbleibendes Blut bildet aber wiederum ein Kontaminationsrisiko.

Ein weiterer, in einigen erfindungsgemäßen Ausführungsformen erzielbarer Vorteil besteht darin, dass erstmals ein einfaches und sicheres Verfahren vorgeschlagen wird, mit welchem das Restgewicht nach Beendigung der Blutbehandlung sowohl des Blutfilters als auch des Blutkreislaufs beachtlich verringert werden kann. So wird erfindungsgemäß aufgezeigt, wie sowohl Blutkammer des Blutfilters als auch Blutkreislauf geleert werden, wobei dies in manchen Ausführungsformen ganz oder nahezu vollständig automatisch, jedenfalls aber mit geringer Tätigkeit des medizinischen Personals erfolgen kann. Dies wird nicht zuletzt durch die optimierte zeitliche Abfolge des Entleerens von Blutkammer einerseits und des Entleerens der übrigen, in manchen erfindungsgemäßen Ausführungsformen ebenfalls entleerten Abschnitte des Blutfilters und des Blutkreislaufs andererseits erzielt.

Im Folgenden wird das Verfahren anhand bevorzugter Ausführungsformen desselben unter Bezugnahme auf die angehängte Zeichnung beschrieben. In der Zeichnung gilt:
- **Fig.** 1: zeigt schematisch vereinfacht Abschnitte einer erfindungsgemäßen, medizinischen Behandlungsvorrichtung mit einer Blutkassette zur Durchführung des Verfahrens in einer ersten beispielhaften Ausführungsform;
- **Fig. 2**: zeigt die medizinische Behandlungsvorrichtung der Fig. 1 bei der Durchführung des Verfahrens in einer zweiten beispielhaften Ausführungsform; und
- **Fig. 3**: zeigt die gemessene Transmission (in Prozent (%)), aufgetragen über dem Reinfusionsvolumen (in Milliliter (ml)).

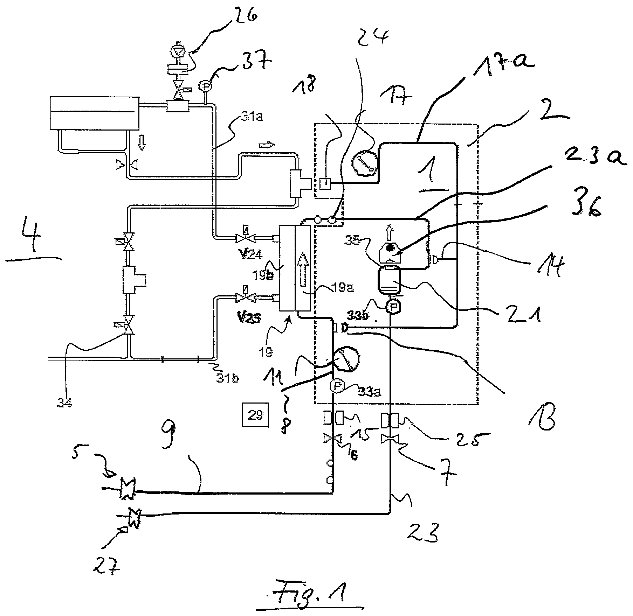

Fig. 1 zeigt einen extrakorporalen Blutkreislauf 1, welcher mittels Double-Needle-Zugangs mit dem Gefäßsystem des nicht dargestellten Patienten verbunden ist. Der Blutkreislauf 1 liegt in Abschnitten hiervon in oder auf einer Blutkassette 2 (beispielsweise wie oben beschrieben) vor. Er ist mit einer Behandlungsvorrichtung 4 verbunden.

### Der Blutkreislauf 1 weist eine arterielle

Patientenschlauchklemme 6 und eine arterielle Konnektionsnadel 5 (als Beispiel einer Zugangseinrichtung) eines arteriellen Abschnitts oder einer arteriellen Patientenleitung oder Blutleitung 9 auf. Der Blutkreislauf 1 weist ferner eine venöse Patientenschlauchklemme 7 und eine venöse Konnektionsnadel 27 (als Beispiel einer weiteren oder zweiten Zugangseinrichtung) eines venösen Abschnitts oder einer venösen Patientenleitung oder Blutleitung 23 auf.

Eine Blutpumpe 11 ist im arteriellen Abschnitt 9 vorgesehen, eine Substituatpumpe 17 ist mit einer Substituatleitung 17a verbunden. Die Substituatleitung 17a kann mittels eines, vorzugsweise automatischen, Substituatports 18 mit einer Substituatquelle verbunden werden. Mittels der Substituatpumpe 17 kann Substituat per Prädilution oder per Postdilution über zugehörige Leitungen 13 bzw. 14 in Leitungsabschnitte, beispielsweise in den arteriellen Abschnitt 9 bzw. in einen venösen Abschnitt 23a (zwischen einer Blutkammer 19a und einer Single-Needle-Kammer 36, siehe unten) des Blutkreislaufs 1 eingebracht werden.

In den Blutkreislauf 1 ist ein Blutfilter 19 eingeschaltet. Dieser weist eine mit dem arteriellen Abschnitt 9 und mit dem venösen Abschnitt 23 verbundene Blutkammer 19a auf. Eine Dialysatkammer 19b des Blutfilters 19 ist mit einer zur Dialysatkammer 19b führenden Dialysierflüssigkeitszulaufleitung 31a und einer von der Dialysatkammer 19b fortführenden Dialysatablaufleitung 31b verbunden.

Die Dialysierflüssigkeitszulaufleitung 31a weist optional ein Ventil V24 auf, mittels welchem der Fluss innerhalb der Dialysierflüssigkeitszulaufleitung 31a unterbunden werden kann. Die Dialysatablaufleitung 31b weist optional ein Ventil V25 auf, mittels welchem der Fluss innerhalb der Dialysatablaufleitung 31b unterbunden werden kann.

Die Dialysierflüssigkeitszulaufleitung 31a ist ferner optional mittels eines weiteren, maschineninternen Ventils mit einer Druckluftquelle 26 verbunden. Die Druckluftquelle 26 kann Bestandteil der Behandlungsvorrichtung 4 sein oder getrennt hiervon vorliegen. Stromab der Druckluftquelle 26 kann ein Drucksensor 37 vorgesehen sein.

Zur Durchführung des Verfahrens mit dem Ziel, die Blutkammer 19a des Blutfilters 19 nach Ende der Behandlung zu leeren, kann die arterielle Patientenschlauchklemme 6 geschlossen, die venöse Patientenschlauchklemme 7 geöffnet und/oder die Blutpumpe 11 (exemplarisch als Rollenpumpe ausgebildet) angehalten werden und Substituat oder Dialysierflüssigkeit mittels der Substituatpumpe 17 über die Zugabestelle 13 in Prädilution dem Blutkreislauf 1 und der Blutkammer 19a zugeführt werden.

Alternativ, oder ergänzend, kann das Substituat nicht mittels der Substituatpumpe 17 sondern durch Betreiben der Blutpumpe 11 eingebracht werden. Dazu wird die arterielle Patientenschlauchklemme 6 geschlossen und Substituat über eine Zuleitung 8 aus einem Vorratsbehälter für das Substituat in den extrakorporalen Blutkreislauf 1 eingebracht.

Der entstandene Substituat-Blut-Inhalt wird durch Betreiben der Blutpumpe 11 und/oder der Substituatpumpe 17 entlang des Leitungsinneren des extrakorporalen Blutkreislaufs 1 gefördert und durch den Blutfilter 19, eine venöse Luftabscheidekammer 21 und einen venösen Abschnitt 23 des extrakorporalen Blutkreislaufs 1 gedrückt bzw. gefördert, um so das Blut aus dem extrakorporalen Blutkreislauf 1 in Richtung zur venösen Konnektionsnadel 27 aus dem Blutfilter 19 zu entfernen.

Im venösen Abschnitt 23 des extrakorporalen Blutkreislaufs 1 ist optional ein venöser Substituat-Blut-Detektor 25 als ein weiteres Beispiel einer Erfassungseinrichtung angeordnet, der das Auftreten von Substituat an einer vorbestimmten Position des Leitungsinneren des extrakorporalen Blutkreislaufs 1 erkennt. Die Blutpumpe 11 und/oder die Substituatpumpe 17 fördert den Substituat-Blut-Inhalt solange weiter, bis das Blut im venösen Abschnitt 23 des extrakorporalen Blutkreislaufs 1 aus selbigem entfernt und über die venöse Konnektionsnadel 27 in das Gefäßsystem des Patienten rückgeführt worden ist und/oder bis am venösen Substituat-Blut-Detektor 25 das Auftreten von Subsituat im Leitungsinneren erfasst wird. Die Förderleistung aller Pumpen wird beendet. Es kann ein optisches und/oder akustisches Signal ausgegeben werden.

Die Steuerung oder Regelung der Behandlungsvorrichtung 4 kann mittels einer Steuerungs- oder Regelungseinrichtung 29 erfolgen.

Die Anordnung der Fig. 1 umfasst einen optionalen Detektor 15 zum Detektieren von Luft und/oder Blut. Die Anordnung der Fig. 1 umfasst ferner einen oder zwei Drucksensoren 33a, 33b an den in Fig. 1 gezeigten Stellen.

Das eingebrachte Substituat kann in einem Pufferbehälter zwischengespeichert werden, aus welchem es unter einem geringeren Fluss als jenem entlassen wird, mit welchem es in den Pufferbehälter eingebracht wurde.

Als Pufferbehälter kommt in der Anordnung der Fig. 1 insbesondere die Single-Needle-Kammer 36 in Frage. Diese kann durch entsprechendes Schalten eines optionalen Single-Needle-Ventils 35, welches die Single-Needle-Kammer 36 vorzugsweise von weiteren, insbesondere von allen weiteren blutführenden Strukturen trennt, in den Blutkreislauf eingeschaltet werden oder nicht. Wird die Single-Needle-Kammer 36 durch Öffnen des Single-Needle-Ventils 35 für Blut oder anderes Fluid, das aus dem venösen Abschnitt 23a stromabwärts des Blutfilters 19 strömt, zugänglich, und wird zugleich beispielsweise mittels Schließens der venösen Patientenschlauchklemme 7 ein Vorbeiströmen des Bluts oder Fluids an der Single-Needle-Kammer 36 verhindert, so kann Blut zunächst in der Single-Needle-Kammer 36 gepuffert oder gespeichert werden, bevor dieses nach Öffnen der venösen Patientenschlauchklemme 7 mit gewünschtem Fluss die Single-Needle-Kammer 36 in Richtung zur venösen Patientenschlauchklemme 7 wieder verlässt.

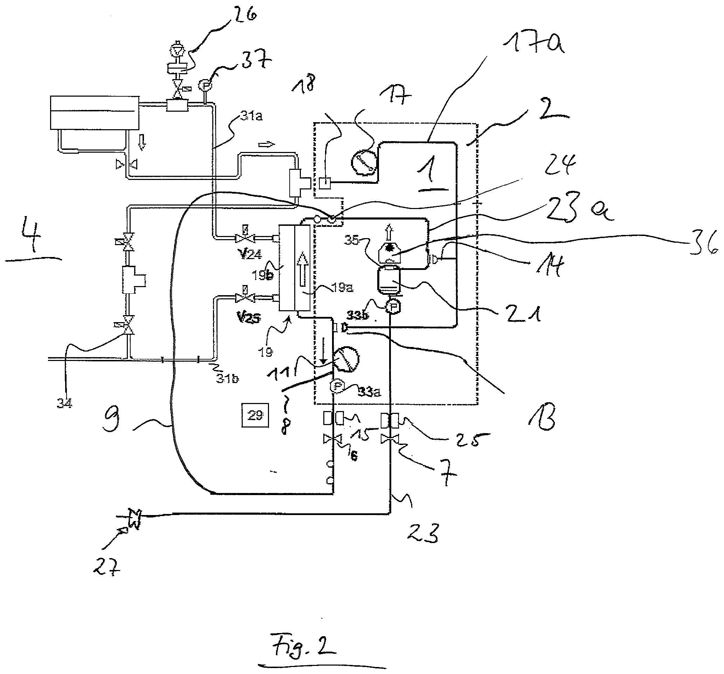

Alternativ kann exemplarisch wie folgt vorgegangen werden, wie **Fig. 2** zeigt:
Der extrakorporale Blutkreislauf 1 wird vom Gefäßsystem des Patienten dekonnektiert, beispielsweise indem die arterielle Konnektionsnadel 5 aus dem Arm des Patienten gezogen wird. Jedenfalls aber wird die arterielle Blutleitung 9 stromabwärts des Blutfilters 19 an einer Verbindungsstelle 24 mit dem venösen Abschnitt 23a verbunden. Die arterielle Patientenschlauchklemme 6 bleibt geöffnet, und aufgrund des Drucks der Substituatpumpe 17 wird Substituat entlang der Richtung der beiden Pfeile sowohl durch die Blutkammer 19a als auch durch die arterielle Blutleitung 9 gefördert. Das in Blutkammer 19a als auch in der arteriellen Blutleitung 9 vorhandene Blut wird auf diese Weise sowohl aus Blutkammer 19a als auch aus der arteriellen Blutleitung 9 entfernt.

In den Fig. 1 und 2 kommt die Single-Needle-Kammer 36 als Pufferbehälter zum Einsatz, insbesondere bei oder nach einem Double-Needle-Verfahren, bei welchem der Patient mittels zweier Blutleitungen 9, 27 mit dem extrakorporalen Blutkreislauf 1 verbunden ist. Für den Fachmann ist aber erkennbar, dass die vorliegende Erfindung auch ohne Verwendung eines Pufferbehälters erfolgen kann, wie vorstehend beschrieben ist. Zudem kann auch jeder andere Pufferbehälter in Betracht gezogen werden.

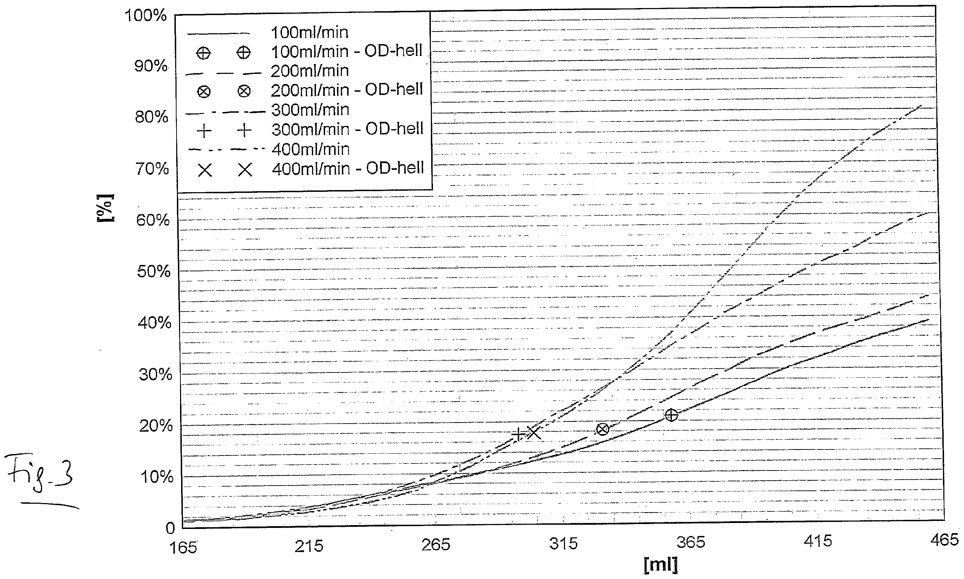

**Fig. 3** zeigt die gemessene Transmission (in %) über dem Reinfusionsvolumen (in ml), welche von den Erfindern in einem Versuchsaufbau unter Verwendung eines FX_{hdf}1000-Dialysators aus dem Hause der Anmelderin bei unterschiedlichen Substituatflussraten, wie in der Graphik der Fig. 3 angegeben, gemessen wurde.

Beim Versuchsaufbau wurde Substituat arteriell angesaugt. An der venösen Blutleitung 23, siehe Fig. 1 oder 2, wurde ca. 10 cm unterhalb bzw. stromab des venösen optischen Detektors 25 und der venösen Patientenschlauchklemme 7 ein Photometer angebracht, welches eine transmittierte Lichtleistung ermittelt. Normiert war der Sensor des Photometers auf klares, blutfreies Substituat. Die Transmission wurde in Abhängigkeit der Spülmenge bei unterschiedlichen Substituatflüssen erfasst. Der Hämatokrit des verwendeten Rinderblutes beträgt vor jedem Versuch ca. 30 %.

Fig. 3 zeigt die vorteilhafte Effizienz mittels des Verfahrens, bei welchem höhere Substituatflüsse zum Spülen als üblich zum Einsatz kommen. Man erkennt, dass mit steigendem Substituatfluss als Spülfluss der Leitungsinhalt früher klar wird bzw. die gemessene Transmission zunimmt.

Bei vorbestimmtem oder festem Reinfusionsvolumen sinkt die im extrakorporalen Blutkreislauf verbleibende Menge an Blut. Die in den Graphen jeweils markierten Punkte bezeichnen das Reinfusionsvolumen, bei welchem die Erfassungsvorrichtung, z. B. der optische Sensor, kein Blut mehr erkennt.

Die vorliegende Erfindung ist nicht auf die vorstehend beschriebenen Ausführungsformen beschränkt, diese dienen lediglich der Veranschaulichung. Auch ist die Erfindung nicht auf ein Entleeren des Inhalts oder Teile hiervon bei bestehender Konnektierung mit dem Gefäßsystem beschränkt.

### Bezugszeichenliste

- 1: extrakorporaler Blutkreislauf
- 2: Blutkassette
- 4: Behandlungsvorrichtung
- 5: Zugangseinrichtung, beispielsweise arterielle Konnektionsnadel
- 6: arterielle Patientenschlauchklemme
- 7: venöse Patientenschlauchklemme
- 8: Zuleitung
- 9: arterieller Abschnitt oder arterielle Blutleitung oder arterielle Patientenleitung
- 11: Blutpumpe
- 13: Zugabestelle für Substituat (Prädilution)
- 14: Zugabestelle für Substituat (Postdilution)
- 15: arterieller Luft-Blut-Detektor
- 17: zweite Fördereinrichtung, beispielsweise eine Substituatpumpe
- 17a: Substituatleitung
- 18: automatischer Substituatport
- 19: Blutfilter
- 19a: Blutkammer
- 19b: Dialysatkammer
- 21: venöse Luftabscheidekammer
- 23: venöser Abschnitt oder venöse Blutleitung
- 23a: venöser Abschnitt
- 24: Verbindungsstelle
- 25: venöser Substituat-Blut-Detektor
- 26: Druckluftquelle
- 27: Zugangseinrichtung, beispielsweise venöse Konnektionsnadel
- 29: Steuerungs- oder Regelungseinrichtung
- 31a: Dialysierflüssigkeitszulaufleitung
- 31b: Dialysatablaufleitung
- 33a, b: Drucksensoren
- 35: Single-Needle-Ventil
- 36: Single-Needle-Kammer
- 37: Drucksensor
- V24: Ventil
- V25: Ventil

## Patentansprüche

1. Steuerungs- und/oder Regelungseinrichtung (29) konfiguriert, um ein Verfahren zum Entfernen von Fluid aus einem zur Blutbehandlung eines Patienten verwendeten Blutfilter (19) oder Blutkreislauf (1) nach Beendigung der Blutbehandlungssitzung im Zusammenwirken mit einer medizinischen Blutbehandlungsvorrichtung (4) durchzuführen,
wobei der Blutfilter (19) eine Blutkammer (19a) und eine Dialysatkammer (19b) aufweist, zwischen welchen eine Membran angeordnet ist, wobei die Blutkammer (19a) zur Blutbehandlung mit einer zur Blutkammer (19a) führenden arteriellen Blutleitung (9) und einer von der Blutkammer (19a) wegführenden venösen Blutleitung (23) sowie einer zur Dialysatkammer (19b) führenden Dialysierflüssigkeitszulaufleitung (31a) und einer von der Dialysatkammer (19b) wegführenden
Dialysatablaufleitung (31b) verbunden ist, **dadurch gekennzeichnet, dass** das Verfahren den Schritt umfasst:
- Verdrängen des Fluids aus der Blutkammer (19a) durch Einbringen von Substituat oder Dialysierflüssigkeit in die arterielle Blutleitung (9) mit einem Fluss, welcher, zumindest phasenweise, über dem mittleren und/oder über dem maximalen Substituatfluss der vorangegangenen Blutbehandlung liegt; und
- Begrenzen der mittleren Reinfusionsgeschwindigkeit mittels Niedrigflussphasen, welche auf Hochflussphasen folgen, zum Erzielen eines gewünschten Mittelwerts eines Reinfusionsvolumenstroms.

2. Steuerungs- und/oder Regelungseinrichtung (29) nach Anspruch 1, wobei das Verfahren den Schritt umfasst:
- Verdrängen des Fluids aus der Blutkammer (19a) durch Einbringen von Substituat oder Dialysierflüssigkeit in die arterielle Blutleitung (9) mit einem Fluss, welcher, zumindest phasenweise, über 150 ml/min, über 200 ml/min, über 250 ml/min, über 300 ml/min, über 350 ml/min, über 400 ml/min, über 450 ml/min, über 500 ml/min, über 550 ml/min und/oder gleich 600 ml/min liegt.

3. Steuerungs- und/oder Regelungseinrichtung nach einem der Ansprüche 1 bis 2, wobei zum Verdrängen des Fluids aus der Blutkammer (19a) eingebrachtes Substituat oder eingebrachte Dialysierflüssigkeit mit einem ersten Fluss in einen Pufferbehälter hinein und mit einem zweiten Fluss aus dem Pufferbehälter herausgeführt wird, wobei der erste Fluss höher ist als der zweite.

4. Steuerungs- und/oder Regelungseinrichtung nach Anspruch 3, wobei der Pufferbehälter stromabwärts der Blutkammer (19a) angeordnet ist.

5. Steuerungs- und/oder Regelungseinrichtung nach einem der Ansprüche 3 und 4, wobei der Pufferbehälter eine Single-Needle-Kammer (36) einer Blutkassette (2) ist.

6. Steuerungs- und/oder Regelungseinrichtung (29) nach einem der vorangegangenen Ansprüche, konfiguriert, um im Zusammenwirken mit einer medizinischen Blutbehandlungsvorrichtung (4) eine Blutbehandlung und eine sich anschließende Verdrängung des Fluids aus der Blutkammer (19a) durch eingebrachtes Substituat mit einem vorbestimmten Fluss an Substituat durch den Blutfilter (19) zu bewirken, welcher einen Fluss in den Patienten bewirkt, welcher im Mittel 150 ml/min, vorzugsweise zwischen 135 ml/min und 165 ml/min, beträgt.

7. Medizinische Behandlungsvorrichtung (4) mit
- wenigstens einem extrakorporalen Blutkreislauf (1) mit einem Leitungsinneren;
- wenigstens einer an oder in dem extrakorporalen Blutkreislauf (1) angeordneten Blutpumpe (11) zum Fördern von Blut innerhalb des Leitungsinneren des extrakorporalen Blutkreislaufs (1);
- wenigstens einer Einrichtung zum Einbringen von Substituat oder Dialysierflüssigkeit in die arterielle Blutleitung (9);
**gekennzeichnet durch**
- wenigstens eine Steuerungs- und/oder Regelungseinrichtung (29) nach einem der Ansprüche 1 bis 6.

8. Medizinische Behandlungsvorrichtung (4) nach Anspruch 7, welche einen Pufferbehälter zum Aufnehmen von Fluid, welches aus der Blutkammer (19a) verdrängt wurde, aufweist oder hiermit, vorzugsweise in Fluidkommunikation, verbunden ist.

9. Medizinische Behandlungsvorrichtung (4) nach einem der Ansprüche 7 oder 8, wobei der Pufferbehälter stromabwärts der Blutkammer (19a) angeordnet ist.

10. Medizinische Behandlungsvorrichtung (4) nach einem der Ansprüche 7 bis 9, verbunden mit einer Blutkassette (2), wobei der Pufferbehälter eine Single-Needle-Kammer (36) der Blutkassette (2) ist.

11. Digitales Speichermedium, insbesondere in Form einer Diskette, CD oder DVD oder EPROM, mit elektronisch auslesbaren Steuersignalen, konfiguriert, um derart mit einem programmierbaren Computersystem zusammenzuwirken, dass die in einem der Ansprüche 1, 2, 3 und/oder 6 genannten maschinellen Schritte des Verfahrens veranlasst werden.

12. Computerprogramm-Produkt mit einem auf einem maschinenlesbaren Träger gespeicherten Programmcode zur Veranlassung der in einem der Ansprüche 1, 2, 3 und/oder 6 genannten maschinellen Schritte, wenn das Computerprogramm-Produkt auf einem Rechner abläuft.

13. Computerprogramm mit einem Programmcode zur Veranlassung der in einem der Ansprüche 1, 2, 3 und/oder 6 genannten maschinellen Schritte, wenn das Computerprogramm auf einem Computer abläuft.

## Claims

1. A control and/or regulating device (29) configured to perform a method for removing fluid from a blood filter (19) or a blood circuit (1) used for blood treatment of a patient after the end of the blood treatment session in cooperation with a medical blood treatment apparatus (4),
wherein the blood filter (19) comprises a blood chamber (19a) and a dialysate chamber (19b), between which a membrane is arranged, the blood chamber (19a) for blood treatment being connected with an arterial blood line (9) leading to the blood chamber (19a) and with a venous blood line (23) leading away from the blood chamber (19a), as well as with a dialysis liquid supply line (31a) leading to the dialysate chamber (19b) and a dialysate draining line (31b) leading away from the dialysate chamber (19b),
**characterized in that** the method comprises the steps of:
- evacuating the fluid from the blood chamber (19a) by introducing substitute or dialysis liquid into the arterial blood line (9) with a flow which is, at least at times, above the average and/or above the maximum substitute flow of the previous blood treatment; and
- limiting the average reinfusion speed by means of low-flow phases which follow high-flow phases in order to achieve a desired average value of a reinfusion volume flow.

2. The control and/or regulating device (29) according to claim 1, wherein the method comprises the step of:
- evacuating the fluid from the blood chamber (19a) by introducing substitute or dialysis liquid into the arterial blood line (9) with a flow which is, at least at times, above 150 ml/min, above 200 ml/min, above 250 ml/min, above 300 ml/min, above 350 ml/min, above 400 ml/min, above 450 ml/min, above 500 ml/min, above 550 ml/min, and/or equal to 600 ml/min.

3. The control and/or regulating device according to anyone of the claims 1 to 2, wherein to evacuate the fluid from the blood chamber (19a), introduced substitute or introduced dialysis liquid is led, with a first flow into a buffer container and, with a second flow, out of the buffer container, the first flow being higher than the second flow.

4. The control and/or regulating device according to claim 3, wherein the buffer container is arranged downstream of the blood chamber (19a).

5. The control and/or regulating device according to anyone of the claims 3 and 4, wherein the buffer container is a single needle chamber (36) of a blood cassette (2).

6. The control and/or regulating device (29) according to anyone of the preceding claims, configured to, in cooperation with a medical blood treatment apparatus (4), effect a blood treatment and a subsequent evacuation of the fluid from the blood chamber (19a) by introduced substitute with a predetermined flow of substitute through the blood filter (19), which causes a flow into the patient, which amounts on average to 150 ml/min, preferably between 135 ml/min and 165 ml/min.

7. A medical treatment apparatus (4) comprising:
- at least one extracorporeal blood circuit (1) with a line interior;
- at least one blood pump (11) arranged on or in the extracorporeal blood circuit (1) for conveying blood within the line interior of the extracorporeal blood circuit (1);
- at least one device for introducing substitute or dialysis liquid into the arterial blood line (9);
**characterized by**:
- at least one control and/or regulating device (29) according to anyone of claims 1 to 6.

8. The medical treatment apparatus (4) according to claim 7, which comprises or is connected, preferably in fluid communication, with a buffer container for receiving fluid that has been evacuated from the blood chamber (19a).

9. The medical treatment apparatus (4) according to anyone of claims 7 or 8, wherein the buffer container is arranged downstream of the blood chamber (19a).

10. The medical treatment apparatus (4) according to anyone of claims 7 to 9, connected to a blood cassette (2), wherein the buffer container is a single-needle chamber (36) of the blood cassette (2).

11. A digital storage medium, in particular in the form of a floppy disk, CD or DVD or EPROM, with electronically readable control signals, configured to interact with a programmable computer system so as to initiate the mechanical steps of the method mentioned in anyone of claims 1, 2, 3 and/or 6.

12. A computer program product comprising a program code stored on a machine-readable support to initiate the mechanical steps mentioned in anyone of claims 1, 2, 3 and/or 6 when the computer program product is running on a computer.

13. A computer program comprising a program code for initiating the mechanical steps mentioned in anyone of claims 1, 2, 3 and/or 6, when the computer program is running on a computer.

## Revendications

1. Un dispositif de contrôle et/ou de régulation (29) configuré pour mettre en œuvre un procédé d'élimination de fluide d'un filtre sanguin (19) ou d'un circuit sanguin (1) utilisé pour le traitement du sang d'un patient à la fin de la séance de traitement du sang, en coopération avec un appareil médical de traitement du sang (4),
où le filtre sanguin (19) comprend une chambre à sang (19a) ainsi qu'une chambre à dialysat (19b), entre lesquelles est agencée une membrane, la chambre à sang (19a) pour le traitement du sang étant reliée à un conduit sanguin artériel (9) menant à la chambre à sang (19a) et à un conduit sanguin veineux (23) s'éloignant de la chambre à sang (19a), ainsi qu'à un conduit d'alimentation en liquide de dialyse (31a) menant à la chambre à dialysat (19b) et à un conduit d'évacuation de dialysat (31b) s'éloignant de la chambre à dialysat (19b),
**caractérisé en ce que** le procédé comprend les étapes consistant à:
- évacuer le fluide de la chambre à sang (19a) en introduisant du substitut ou du liquide de dialyse dans le conduit sanguin artériel (9) avec un débit étant, au moins par moments, supérieur au débit de substitut moyen et/ou supérieur au débit maximal de substitut du traitement du sang précédent; et
- limiter la vitesse moyenne de réinfusion au moyen de phases à faible débit suivant des phases à haut débit pour atteindre une valeur moyenne souhaitée d'un débit volumique de réinfusion.

2. Le dispositif de contrôle et/ou de régulation (29) selon la première revendication, où le procédé comprend l'étape consistant à:
- évacuer le fluide de la chambre à sang (19a) en introduisant du substitut ou du liquide de dialyse dans le conduit sanguin artériel (9) avec un débit étant, au moins par moments, supérieur à 150 ml/min, supérieur à 200 ml/min, supérieur à 250 ml/min, supérieur à 300 ml/min, supérieur à 350 ml/min, supérieur à 400 ml/min, supérieur à 450 ml/min, supérieur à 500 ml/min, supérieur à 550 ml/min, et/ou égal à 600 ml/min.

3. Le dispositif de contrôle et/ou de régulation selon l'une quelconque des revendications 1 à 2, où pour évacuer le fluide de la chambre à sang (19a), le substitut introduit ou le liquide de dialyse introduit est amené avec un premier débit dans un récipient tampon et, avec un second débit hors du récipient tampon, le premier débit étant supérieur au second débit.

4. Le dispositif de contrôle et/ou de régulation selon la revendication 3, où le récipient tampon est agencé en aval de la chambre à sang (19a).

5. Le dispositif de contrôle et/ou de régulation selon l'une quelconque des revendications 3 et 4, où le récipient tampon est une chambre à aiguille unique (36) d'une cassette à sang (2).

6. Le dispositif de contrôle et/ou de régulation (29) selon l'une quelconque des revendications précédentes, configuré pour effectuer, en coopération avec un appareil médical de traitement du sang (4), un traitement du sang ainsi qu'une évacuation subséquente du fluide hors de la chambre à sang (19a) par l'introduction d'un substitut ayant un débit prédéterminé de substitut au travers du filtre sanguin (19), provoquant un écoulement dans le patient étant en moyenne de 150 ml/min, de préférence entre 135 ml/min et 165 ml/min.

7. Un appareil de traitement médical (4) comprenant:
- au moins un circuit sanguin extracorporel (1) avec un intérieur de conduit;
- au moins une pompe à sang (11) agencée sur ou dans le circuit sanguin extracorporel (1) pour acheminer le sang à l'intérieur de l'intérieur de conduit du circuit sanguin extracorporel (1);
- au moins un dispositif pour introduire du substitut ou un liquide de dialyse dans le conduit sanguin artériel (9);
**caractérisé par**
- au moins un dispositif de contrôle et/ou de régulation (29) selon l'une quelconque des revendications 1 à 6.

8. L'appareil de traitement médical (4) selon la revendication 7, qui comprend un récipient tampon ou qui est relié à celui-ci, de préférence en communication fluidique, pour recevoir un fluide qui a été évacué hors de la chambre à sang (19a).

9. L'appareil de traitement médical (4) selon l'une quelconque des revendications 7 ou 8, où le récipient tampon est agencé en aval de la chambre à sang (19a).

10. L'appareil de traitement médical (4) selon l'une quelconque des revendications 7 à 9, relié à une cassette à sang (2), où le récipient tampon est une chambre à aiguille unique (36) de la cassette à sang (2).

11. Un support de stockage numérique, notamment sous forme de disquette, CD ou DVD ou EPROM, avec des signaux de commande lisibles électroniquement, configuré pour interagir avec un système informatique programmable de manière à initier les étapes mécaniques du procédé mentionné dans l'une quelconque des revendications 1, 2, 3 et/ou 6.

12. Un produit de programme informatique comprenant un code de programme stocké sur un support lisible par une machine de manière à initier les étapes mécaniques mentionnées dans l'une quelconque des revendications 1, 2, 3 et/ou 6 lorsque le produit de programme informatique est exécuté sur un ordinateur.

13. Un programme informatique comprenant un code de programme pour initier les étapes mécaniques mentionnées dans l'une des revendications 1, 2, 3 et/ou 6 lorsque le programme d'ordinateur est exécuté sur un ordinateur.
